# EUROPÄISCHE PATENTSCHRIFT

(11) **EP 1 193 320 B1**
(45) Veröffentlichungstag und Bekanntmachung des Hinweises auf die Patenterteilung: **16.03.2005**
(21) Anmeldenummer: 01122702.2
(22) Anmeldetag: 21.09.2001
(51) Int. Cl.: C22C 5/02, A61K 6/04

(54) **Aufbrennfähige, hochgoldhaltige Dentallegierung**
Fireable dental alloy with a high gold content
Alliage dentaire à haute teneur en or et apte au cuisson

(30) Priorität: 29.09.2000 CH 19172000
(43) Veröffentlichungstag der Anmeldung: 03.04.2002
(73) Patentinhaber: Cendres & Metaux SA, 2501 Biel (CH)
(72) Erfinder: Guo-Huang, Kangping, 2074 Marin (CH); Baltzer, Niklaus, 2504 Biel (CH)
(74) Vertreter: AMMANN INGENIEURS-CONSEILS EN PROPRIETE INTELLECTUELLE SA BERNE

(56) Entgegenhaltungen:
- EP-A- 0 904 765
- DE-A- 2 746 525
- DE-A- 2 755 913
- FR-A- 2 218 083

## Beschreibung

Die vorliegende Erfindung bezieht sich auf eine universell einsetzbare Dentallegierung, die hochgoldhaltig und aufbrennfähig ist. Es sind eine grosse Anzahl von aufbrennfähigen, hochgoldhaltigen Dentallegierungen bekannt, so z.B. aus dem Katalog der Anmelderin, Ausgabe 4.99 , worin über ein Dutzend solcher Dentallegierungen unter der Sammelmarke Esteticor® beschrieben und aufgelistet sind.

Aus der FR-A-2 218 083 ist ebenfalls eine Dentallegierung bekannt, die hochgoldhaltig und aufbrennfähig ist.

Die verschiedenen Legierungen haben jedoch verschiedene Indikationsbereiche und es hat sich davon ausgehend der Wunsch und damit auch die Aufgabe herauskristallisiert, eine hochgoldhaltige Dentallegierung bereitzustellen, die in einem weiten Anwendungsgebiet von Inlay bis grossen Brücken anwendbar ist und bessere mechanische Eigenschaften, insbesondere eine höhere Festigkeit - nach dem Aufbrennen von herkömmlichen hoch schmelzenden Keramiken, aber auch für Keramiken mit reduzierter Brenntemperatur und normaler Expansion - aufweist als die vorbekannten Dentallegierungen. Diese Aufgabe wird mit der Legierung gemäss Patentanspruch 1 gelöst. Falls an die Dentallegierung hohe Anforderungen bezüglich Biokompatibilität gestellt werden, ist die Dentallegierung gemäss Patentanspruch 2 bevorzugt.

Die Erfindung wird nun im Folgenden anhand von Ausführungsbeispielen näher erläutert werden. Wie aus der Fülle vorbekannter hochgoldhaltiger Dentallegierungen zu entnehmen ist und aus Versuchen, anhand von Büchern und dergleichen für einen Fachmann bekannt ist, können einzelne Elemente, neben den Edelmetallanteilen Gold und Platin, gewisse Effekte hervorrufen und es ist daher bekannt, das beispielsweise Silber die Liquidität der Legierung erhöht oder Zink eine Legierung härten kann oder Iridium als Kornverfeinerer eingesetzt werden kann. Das Gleiche gilt für die zusätzlichen Metalle wie Rhodium, Ruthenium und dergleichen.

Langwierige Versuche haben nun überraschend ergeben, dass die nachfolgend beschriebene Legierung innerhalb der angegebenen Grenzen eine gelbe, aufbrennfähige Legierung mit sehr guten mechanischen Eigenschaften ergibt, wobei sich diese Legierung aus 80, 0 - 84,9 % Gold, 7,1 - 13,0 % Platin, 0,1 - 8,0 % bevorzugt 1, 2 - 8,0 % Palladium, 0 - 1,2 % Silber, 0,7 - 3,5 % Zink, 0 - 1,0 % Eisen, Iridium, Ruthenium, Rhodium, Tantal, Mangan, Rhenium, Niob, 0 - 3,5 % Zinn, Indium, Gallium oder 0 - 0,5 % Kupfer zusammensetzt, wobei die Prozentangaben Gewichtsprozente sind.

Innerhalb der angegebenen Grenzen haben Versuchsreihen ergeben, dass eine bevorzugte Dentallegierung, die ausserdem höchste Biokompatibilitätsanforderungen erfüllt sich aus 84,0 % Gold, 10,9 % Platin, 2,4 % Palladium, 0,2 % Silber, 2,2 % Zink, 0,2 % Eisen und 0,1 % Iridium zusammensetzt. Weitere gute Legierungen ergaben sich mit der Zusammensetzung 84,0 % Gold, 8,9 % Platin, 4,0 % Palladium, 0,5 % Silber, 2,0 % Zink, 0,2 % Eisen, 0,1 Iridium sowie 0,3 % Zinn; oder 82,0 % Gold, 8,9 % Platin, 6,0 % Palladium, 0,5 % Silber, 1,5 % Zink, 0,2 % Eisen, 0,1 % Iridium sowie 0,3 % Tantal und 0,5 % Zinn; oder 82,1 % Gold, 10,9 % Platin, 4,0 % Palladium, 0,2 % Silber, 2,5 % Zink, 0,2 % Eisen, 0,1 % Iridium.

Aus dem Vorgehenden geht hervor, dass ein bevorzugter Bereich, in dem Legierungen mit besonders guten mechanischen Eigenschaften hergestellt werden können sich wie folgt zusammensetzt: 82,0 - 84,0 % Gold, 8.9 - 10,9 % Platin, 4,0 - 6,0 % Palladium, 0,2 - 0,5 % Silber, 1,5 - 2,5 % Zink, 0,2 % Eisen, 0,1 % Iridium und 0- 0,3 % Tantal, 0 - 0,5% Zinn.

Für bestimmte Anwendungen kann es zweckmässig sein, weitere Elemente wie Ruthenium, Rhodium, usw., siehe weiter oben, in einem Bereich von 0 - 1,0 % beizufügen, wobei dies jedoch mit Inkaufnahme von herabgesetzter Biokompatibilität geschehen kann. Sinngemäss können auch Legierungen ausserhalb des bevorzugten, jedoch innerhalb des insgesamt beanspruchten Bereichs verwendet werden, falls ganz bestimmte Eigenschaften hervorgehoben werden sollen.

Zusammenfassend kann gesagt werden, dass die vorgeschlagenen Legierungen
a) der Norm ISO 9693:1999 entsprechen, d.h. einer Dentallegierung, die mit einer Keramik mit niedrigem Wärmeausdehnungskoeffizienten verblendet wird,
b) sehr gute mechanische Eigenschaften, insbesondere eine grosse Festigkeit aufweisen und dies selbst nach dem Aufbrennen der beiden Keramikmassentypen (hoch schmelzend oder mit reduzierter Brenntemperatur und mit normalem Wärmeausdehnungskoeffizient) erreichbar ist.
c) eine hohe mechanische Festigkeit aufweisen,
d) eine gute Biokompatibilität aufweisen, sofern auf die Elemente Kupfer und Indium verzichtet wird, wie dies ohnehin für den Hauptanwendungszweck vorgesehen ist,
e) einen geringen Palladiumanteil aufweisen, und
f) ästhetischen Eigenschaften haben.

## Patentansprüche

1. Hochgoldhaltige, aufbrennfähige entallegierung, **dadurch gekennzeichnet, dass** sie aus 80,0 - 84,9 % Gold, 7,1 - 13,0 % Platin, 0,1 - 8,0 % Palladium, 0 - 1,2 % Silber, 0,7 - 3,5 % Zink, 0 - 1,0 % Eisen, Iridium, Ruthenium, Rhodium, Tantal, Mangan, Rhenium, Niob, 0 - 3,5 % Zinn, Indium, Gallium oder 0 - 0,5 % Kupfer besteht, wobei die Prozentangaben Gewichtsangaben sind.

2. Dentallegierung gemäss Anspruch 1, **dadurch gekennzeichnet dass** der Palladiumgehalt 1,2 - 8,0 % beträgt

3. Dentallegierung nach Anspruch 1 oder 2, **dadurch gekennzeichnet, dass** sie aus 82,0 - 84,0 % Gold, 8.9 - 10,9 % Platin, 4,0 - 6,0 % Palladium, 0,2 - 0,5 % Silber, 1,5 - 2,5 % Zink, 0,2 % Eisen, 0,1 % Iridium und 0- 0,3 % Tantal, 0 - 0,5% Zinn besteht

4. Dentallegierung nach Anspruch 1 oder 2, **dadurch gekennzeichnet, dass** sie aus 84,0 % Gold, 10,9 % Platin, 2,4 % Palladium, 0,2 % Silber, 2,2 % Zink, 0,2 % Eisen und 0,1 % Iridium besteht.

## Claims

1. High gold ceramic-fused-to-metal dental alloy, **characterised in that** it consists of 80.0 to 84.9 % gold, 7.1 to 13.0 % platinum, 0.1 to 8.0 % palladium, 0 to 1.2 % silver, 0.7 to 3.5 % zinc, 0 to 1.0 % iron, iridium, ruthenium, rhodium, tantalum, manganese, rhenium, niobium, 0 to 3.5 % tin, indium, gallium or 0 to 0.5 % copper, the percentages being percentages by weight.

2. Dental alloy according to claim 1, **characterised in that** the palladium content is comprised between 1.2 and 8.0 %.

3. Dental alloy according to claim 1 or 2, **characterised in that** it consists of 82.0 to 84.0 % gold, 8.9 to 10.9 % platinum, 4.0 to 6.0 % palladium, 0.2 to 0.5 % silver, 1.5 to 2.5 % zinc, 0.2 % iron, 0.1 % iridium, and 0.3 % tantalum, 0 to 0.5 % tin.

4. Dental alloy according to claim 1 or 2, **characterised in that** it consists of 84.0 % gold, 10.9 % platinum, 2.4 % palladium, 0.2 % silver, 2.2 % zinc, 0.2 % iron, 0.1 % iridium.

## Revendications

1. Alliage dentaire cérame-métallique à haute teneur en or, **caractérisé en ce qu'**il est composé de 80,0 à 84,9 % d'or, de 7,1 à 13,0 % de platine, de 0,1 à 8,0 % de palladium, de 0 à 1,2 % d'argent, de 0,7 à 3,5 % de zinc, de 0 à 1,0 % de fer, iridium, ruthénium, rhodium, tantale, manganèse, rhénium, niobium, de 0 à 3,5 % d'étain, indium, gallium ou de 0 à 0,5 % de cuivre, les pourcentages étant des pourcentages en poids.

2. Alliage dentaire selon la revendication 1, **caractérisé en ce que** la teneur en palladium est comprise entre 1,2 et 8,0 %.

3. Alliage dentaire selon la revendication 1 ou 2, **caractérisé en ce qu'**il est composé de 82,0 à 84,0 % d'or, de 8,9 à 10,9 % de platine, de 4,0 à 6,0 % de palladium, de 0,2 à 0,5 % d'argent, de 1,5 à 2,5 % de zinc, de 0,2 % de fer, de 0,1 % d'iridium et de 0 à 0,3 % de tantale, 0 à 0,5 % d'étain.

4. Alliage dentaire selon la revendication 1 ou 2, **caractérisé en ce qu'**il est composé de 84,0 % d'or, 10,9 % de platine, de 2,4 % de palladium, de 0,2 % d'argent, de 2,2 % de zinc, de 0,2 % de fer, de 0,1 % d'iridium.
